(19) Europäisches Patentamt
European Patent Office
Office européen des brevets

(11) Numéro de publication : 0 340 054 B1

# (12) FASCICULE DE BREVET EUROPEEN

(45) Date de publication du fascicule du brevet : **27.01.93 Bulletin 93/04**

(51) Int. Cl.$^5$ : **B01F 17/00,** C08L 95/00

(21) Numéro de dépôt : **89400857.2**

(22) Date de dépôt : **28.03.89**

## (54) Compositions cationactives; application aux émulsions bitumineuses.

Jointe à la demande no. 89904093.5/0374205 (numéro de dépôt/numéro de publication de la demande europöenne) par décision du 19.11.90.

(30) Priorité : **30.03.88 FR 8804166**

(43) Date de publication de la demande : **02.11.89 Bulletin 89/44**

(45) Mention de la délivrance du brevet : **27.01.93 Bulletin 93/04**

(84) Etats contractants désignés : **AT BE CH DE ES FR GB GR IT LI LU NL SE**

(56) Documents cités :
**EP-A- 0 051 030**
**EP-A- 0 111 983**
**FR-A- 1 266 909**

(56) Documents cités :
**FR-A- 1 462 981**
**FR-A- 1 554 241**
**FR-A- 2 295 786**
**US-A- 3 975 295**
**US-A- 4 313 895**

(73) Titulaire : **CECA S.A.**
**4 - 8, Cours Michelet La Défense 10**
**F-92800 Puteaux (FR)**

(72) Inventeur : **Brouard, René Auguste Louis**
**87, rue Saint Lazare**
**F-95290 l'Isle-Adam (FR)**
Inventeur : **Navascues, Luc Etienne André**
**31, rue Bayen**
**F-75017 Paris (FR)**

(74) Mandataire : **Haicour, Philippe**
**ELF ATOCHEM S.A., D.P.I. 4-8, Cours Michelet**
**La Défense 10 - Cédex 42**
**F-92091 Paris La Defence (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

### 1. DOMAINE TECHNIQUE

La présente invention se rapporte à des compositions liquides d'amines grasses et de produits analogues, à propriétés cationactives.

Les amines, représentées par la formule générale $R\text{-}NH_2$, où R est un radical hydrocarboné comportant au moins 10 atomes de carbone, développent outre les propriétés chimiques ordinaires qui caractérisent la classe des amines, et notamment une force basique comparable à celle de l'ammoniac, des propriétés tensioactives, notamment aux interfaces eau/liquide hydrocarboné et des propriétés d'adsorption très marquées sur les surfaces minérales. L'industrie fait largement appel à ces propriétés, par exemple pour la mise en émulsion aqueuse de liquides divers, en particulier les liants noirs pour la construction des routes, l'enrichissement des minerais par flottation des minérais, et d'une façon générale pour le traitement des surfaces minérales les plus diverses comme par exemple développement d'un effet antistatique sur les polymères ou sur les fibres, démouillage des surfaces métalliques et réduction de leur corrosion, dispersion des pigments dans les huiles, hydrophobation des sols, réduction de la capacité d'agglomération des grains d'engrais, etc... (voir l'ouvrage Cationic Surfactants par Eric Jungermann, M. Dekker éditeur, 1970).

En fait, on trouve ces propriétés, qui pour la commodité du langage seront par la suite qualifiées de propriétés cationactives, développées au plus haut niveau dans des composés qui comportent au moins un azote aminé, et au moins un groupe hydrophobe comportant une chaîne hydrocarbonée à au moins 10 atomes de carbone. Ces composés répondent à une formule plus large que la formule-type des amines grasses au sens strict, et que l'on peut représenter par $R\text{-}Z\text{-}NH_2$, où Z est un groupe polaire divalent. A l'énumération de quelques exemples pour le groupe Z, l'homme du métier reconnaîtra sans hésitation des produits très usuels. Avec Z = aminopropyl, ce sont les alkylaminopropylamines, comme la suif-N-propylènediamine très connue de l'industrie routière ; avec Z = oxypropyl, ce sont les alkyloxypropylamines, dites éther-amines, utilisées en flottation ; avec Z = amidoéthyl, ce sont les amidoamines, et leurs produits de cyclisation, les imidazolines, largement utilisées comme dopes d'adhésivité des matériaux pour le revêtement des routes ; etc...

### 2. TECHNIQUE ANTERIEURE

Cependant, du fait de l'existence d'une chaîne longue, nécessaire pour que se développent ces propriétés particulières, ces produits ont un point de fusion relativement élevé, et les produits industriels correspondants ont très généralement une consistance pâteuse à température ambiante. Il s'agit là d'une caractéristique fâcheuse à divers égards.

En premier lieu, les transferts de ces produits sous cette forme sont limités à des manipulations manuelles, au demeurant non exemptes de dangers. Ils sont en effet très aggressifs pour la peau et les muqueuses et non dénués de toxicité générale.

La fusion de ces produits s'opère à des températures relativement basses, et les formes fondues dès 40-60°C sont certes très fluides, donc pompables et dosables avec des dispositifs précis et évitant tout contact corporel. Mais à ces températures, ils émettent souvent des produits volatils caustiques (ammoniac, méthylamine, propylamine, etc...) provenant soit d'impuretés industrielles quasi obligatoires dans ce genre de composés, soit de produits de dégradation dûs à leur chauffage et à leur conservation même de courte durée aux températures auxquelles on les a portés pour qu'ils soient suffisamment liquides. Il faut ajouter à cela que cette fusion à relativement basse température est difficile à réaliser dans la pratique industrielle sans risque de surchauffe, et qu'en tout état de cause, de telles opérations de fusion sont fort longues du fait de la mauvaise capacité d'échange thermique bien connue des composés gras.

On a réalisé des compositions fluides à température ambiante en utilisant des solvants. Mis à part l'effet banal de perte d'efficacité relative par dilution, d'autres désavantages de l'emploi des solvants sont bien connus : les solvants lourds se retrouvent, inutiles ou indésirables, dans les produits finals ; les solvants légers sont, certes, éliminables, mais par là même sources d'inflammabilité et/ou de pollution. De plus, les formulations solvantées de composés cationactifs à longue chaîne n'ont généralement pas un comportement satisfaisant au stockage à basse température, comme on l'exposera bientôt.

Le chimiste sait mettre en oeuvre certaines modifications de ces molécules pour abaisser leur point de fusion. C'est ainsi que l'on a eu recours à l'oxyalkylation des alkylamines et des alkylpropylènediamines, comme il est décrit par exemple dans US-A- 2 930 701, où l'on réalise des compositions concentrées pour shampooings à l'aide de solutions chlorhydriques d'oléylpropylènediamines totalement oxyéthylées. Mais on sait aussi que par oxyalkylation on sacrifie une partie des propriétés cationactives. On y rémédie plus ou moins bien par un choix judicieux de la nature du substituant oxyalkylène et du rapport molaire d'oxyalkylation (voir par exemple, FR-A- 1 462 981 qui divulgue des alkylpropylènediamines oxyethylées sur l'azote interne, FR-A- 1 266 909 qui divulgue des suif- ou soja-propylènediamines oxyéthylées ou encore FR-A-2 492 683 qui divulgue des compositions basées sur les suifpropylènediamines oxypropylées). A vrai dire, ces modifications, même effectuées à bon escient, peuvent concourir à une liquidité acceptable à température ambiante, mais celle-ci ne se maintient pas lors de la conservation à des températures négatives. Or il arrive que ces produits soient stockés en fûts en plein air ou sous abris non climatisés. Il apparaît alors des concrets, qui résistent longtemps au retour de températures plus clémentes. Il en résulte une ségrégation des produits avec des différences importantes de composition entre les produits fondus et les concrets. Ces phénomènes indésirables surviennent également dans les présentations solvantées dont il a été question plus haut, et aussi dans les formulations résultant de l'association d'une amine sous forme liquide avec une amine pâteuse ou solide, comme les compositions divulguées dans US-A- 3 975 295, de suifdiamines avec des amines alkylamines oxyéthylées en présence ou non de solvants alcooliques.
Plus récemment (voir FR-A-2 479 019), on a réussi à abaisser fortement le point de fusion des N-suifpropylènediamines sans altérer sensiblement leur comportement cationactif par méthylation de l'azote secondaire, mais ces N-alkyl-N-méthylpropylènediamines présentent une facheuse aptitude à absorber très rapidement le gaz carbonique et l'eau de l'atmosphère en donnant d'abondants concrets, si bien que ces produits perdent leur liquidité par simple exposition ou manipulation à l'air libre.

3. EXPOSE DE L'INVENTION

La présente invention a pour but de remédier à ces inconvénients. Son objet est de fournir des compositions de dérivés cationactifs aminés non inflammables, qui soient absolument liquides à température ambiante, qui conservent intégralement leur liquidité lorsqu'elles restent indéfiniment exposées à l'atmosphère, qui ne donnent pas lieu à formation de gels, de précipités ou de dépôts lorsqu'elles ont été maintenues de façon prolongée à des températures voisines de zéro degré centigrade ou qui ne donnent pas lieu à formation de tels gels, précipités ou dépôts persistant longtemps par retour à la température ambiante.

La demanderesse a trouvé qu'il était possible de réaliser de telles compositions en associant à des amines, diamines ou polyamines grasses partiellement substituées à l'azote par un oxyde d'alkylène tel que l'oxyde d'éthylène, l'oxyde de propylène, l'oxyde de butylène,(que pour la commodité de l'exposé on appellera simplement par la suite amines oxyalkylées), des alkylétheramines de formule générale

$$R\text{-}O\text{-}(CH_2)_n\text{-}[NH\text{-}(CH_2)_m\text{-}]_p\text{-}NH_2$$

où n est un nombre entier égal à 2 ou 3,
où m est un nombre entier égal à 2 ou 3,
où p est un nombre pouvant prendre toutes valeurs de 0 à 3,
où R est une chaîne alkyle ramifiée, du type

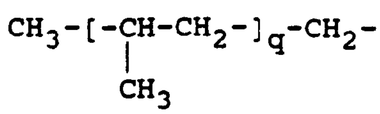

avec q valant de 2 à 5 ; les produits industriels étant souvent des mélanges d'homologues, leur formule centésimale peut s'interpréter par des valeurs de q non entières. (Dans la suite du texte et pour la commodité de l'exposé, on entendra par étheramines, les alkylétheramines au sens qu'on vient de donner ci-dessus).

Dans les compositions selon l'invention, le rapport pondéral entre les amines oxyalkylées et les étheramines est compris entre 20/80 et 80/20. De telles compositions selon l'invention s'obtiennent sans difficulté majeure par simple mélange à température ambiante des composés de type amines oxyalkylées et des composés du type étheramines, l'opération étant d'autant plus aisée que ces substances sont liquides à température ambiante ou tout au moins aisément fusibles à des températures n'excédant pas 50°C, et qu'à l'état liquide, elles sont solubles les unes dans les autres en toutes proportions.

Parmi les étheramines au sens de l'invention, on préfère les alkyloxypropyl-N-propylènediamines qui sont des composés répondant à la formule générale

$$R\text{-}O\text{-}CH_2\text{-}CH_2\text{-}CH_2\text{-}NH\text{-}CH_2\text{-}CH_2\text{-}CH_2\text{-}NH_2$$

Le procédé le plus courant pour leur obtention consiste à condenser un alcool avec le nitrile acrylique, à hydrogéner l'éther-nitrile formé en éthermonoamine, puis à condenser une nouvelle fois ce produit avec le nitrile acrylique et hydrogéner l'étheraminonitrile en étherdiamine. Ce mode de préparation dérive simplement

de celui qu'a divulgué American Cyanamid Co dans "The Chemistry of Acrylonitrile", (NY 1956, pp.216). Pour l'obtention des étheramines selon l'invention, on a recours à des alcools à chaîne ramifiée, industriellement accessibles par hydroformylation d'oligopolypropylènes, en particulier de tétrapropène (voir l'ouvrage Chimie Organique Industrielle, K. Weissermel et H.-J. Arpe, Masson ed., 1981, p. 191). Les composés préférés du type amines oxyakylées selon l'invention sont choisis dans la famille des alkylpropylène diamines oxyalkylées.

On peut les représenter selon la formule générale :

$$R'-NQ_1-CH_2-CH_2-CH_2-NQ_2Q_3$$

où $Q_1$, $Q_2$, $Q_3$ sont l'hydrogène ou un groupe hydroxyéthyle $-CH_2-CH_2-OH$, ou encore un groupe hydroxyéthyloxyéthyle $-CH_2-CH_2-O-CH_2-CH_2-OH$, identiques ou différents, sous la seule condition que ce ne soient pas tous des hydrogènes.

On les obtient de façon très classique par réaction de l'oxyde d'éthylène sur les alkylpropylènediamines correspondantes. On utilise selon l'invention des dérivés munis d'une chaîne alkyle R', linéaire, saturée ou insaturée, comprenant de 10 à 22 atomes de carbone. Dans la pratique, on part de diamines constituées par des mélanges industriels de composés dont les chaînes ont environ 18 atomes de carbone, et qui contiennent une proportion notable de chaînes mono-insaturées ; on les dénomme communément composés oléiques.

4. POSSIBILITES D'APPLICATION INDUSTRIELLE

L'invention a également pour but un procédé pour l'obtention d'émulsions de bitume routier à partir de ces compositions cationactives. L'utilisation des composés dans le procédé de l'invention donne à cette fabrication un caractère nouveau et original, en ce que les émulsifiants peuvent être préparés, stockés et utilisés à température ambiante. Elle permet de régler divers problèmes particulièrement gênants dans cette industrie, notamment ceux de l'automatisation des installations qui réclament des phases liquides, ceux de l'altération physique ou chimique des émulsifiants, tels quels ou formulés, au cours de leur stockage ou de leur manipulation. L'utilisation à température ambiante de phases aqueuses à base des compositions selon l'invention permet également de réussir la mise en émulsion de bitumes durs dans des installations industrielles travaillant à pression atmosphérique, grâce à la réduction de l'apport de calories par la phase aqueuse.

5. EXEMPLES

Les exemples suivants, qui ne sont pas limitatifs feront mieux comprendre l'invention et en montreront les multiples avantages. On y a représenté les composés exemplifiés par des symboles qui sont explicités dans le tableau ci-après. Les produits exemplifiés sont soit des produits issus de fabrications de laboratoire, soit des produits présents sur le marché ; dans ce dernier cas, on a précise les noms commerciaux des produits qui ont été utilisés.

| Symbole | Produit |
|---|---|
| SPDA | Suif propylène diamine (le produit industriel expérimenté est le Dinoram S, de CECA S.A.) |
| OPDA | Oléylpropylène diamine (le produit industriel expérimenté est le Dinoram O, de CECA S.A.) |
| OPDA - 1,5 OE<br>OPDA - 2,0 OE<br>OPDA - 2,5 OE | Oléyl propylène diamines oxyéthylées respectivement à 1,5 ; 2,0 ; 2,5 moles d'oxyde d'éthylène ;<br>(Pour l'OPDA 2,0 OE, on a pris le Dinoramox O 2 OE de CECA S.A.) |
| SPDA - 1,5 OP | Suif propylène diamine oxypropylée à 2 moles d'oxyde de propylène (on a pris le Dinoram SL de CECA S.A.) |
| OPDAM | N-oléyl, N-méthyl, propylène diamine |
| SPDAM | N-suif, N-méthyl, propylène diamine |
| $nC_{13}$ OPPDA | N-tridécyloxypropyl-propylènediamine (on a pris ici un mélange de tridécyl- et pentadécyl-oxypropyl-propylène diamine, de Kenogard $C_{13}$ et $C_{15}$) |
| i $C_{13}$ OPPDA | Isotridécyloxypropyl-propylène-diamine |
| N-ALKIZ | Alkyl-imidazoline-amine (on a pris ici l'Emulsamine L60 de CECA) |

Exemple 1

On compare ici le comportement physique des divers composés et des produits selon l'invention après exposition à des températures basses. On effectue d'une part un essai dit de figeage-défigeage, au cours duquel on soumet le produit contenu dans un tube à essai à un refroidissement intense en le plongeant dans un bain froid d'acétone-carboglace. On suit l'abaissement de température au sein du produit, et on note la température Tf à laquelle le produit se fige. Le tube est alors sorti de son bain froid, et on laisse le produit se réchauffer spontanément à l'ambiance : on note alors la température Tdf à laquelle on observe un début de fusion.

D'autre part, on observe l'aspect du produit conservé dans les conditions normales de température du laboratoire, après exposition de 12 heures à 0° C, et après exposition prolongée de 7 jours à cette température de 0° C.

Dans le tableau suivant, on relate le comportement de quelques compositions cationactives représentatives.

| Produit | % | Essai de Figeage | | Aspect | | |
|---|---|---|---|---|---|---|
| | | Tf | Tdf | Normal | 12 h à 0° | 7 j à 0° |
| OPDA | 100 | + 7 | + 14 | Précipité | Précipité | Précipité |
| $iC_{13}$.OPPDA | 100 | − 28 | − 20 | Fluide | Fluide | Fluide |
| OPDA<br>$iC_{13}$.OPPDA | 50<br>50 | − 8 | − 2 | Précipité | Précipité | Précipité |
| OPDA-1,5.OE | 100 | − 12 | − 6 | Fluide | Fluide | Visqueux |
| OPDA-1,5.OE<br>$iC_{13}$.OPPDA | 50<br>50 | − 21 | − 16 | Fluide | Fluide | Fluide |
| OPDA-2.OE | 100 | − 13 | − 7 | Fluide | Fluide | Visqueux |
| OPDA-2.OE<br>$iC_{13}$.OPPDA | 50<br>50 | − 21 | − 16 | Fluide | Fluide | Fluide |
| OPDA-2,5.OE | 100 | − 11 | − 4 | Fluide | Fluide | Epais |
| OPDA-2,5.OE<br>$iC_{13}$.OPPDA | 50<br>50 | − 19 | − 13 | Fluide | Fluide | Fluide |
| SPDA-2 OP | 100 | + 4 | + 8 | Fluide | Solide | Solide |
| SPDA-2 OP<br>$iC_{13}$.OPPDA | 50<br>50 | − 12 | − 7 | Liquide | Liquide | Liquide |

On constate à la lecture du tableau que seules l'étheramine et les compositions à base d'étheramine et d'amines oxyalkylées, non seulement sont liquides à température ambiante et apparemment liquides à des températures inférieures à 0° C, mais conservent de façon imprévue leur fluidité après exposition prolongée à cette température de 0°C.

Exemple 2

Comportement des produits par exposition à l'atmosphère.

On observe ici la formation de composés croûteux par exposition à l'air. Ces composés sont dûs pour l'essentiel à la formation de carbamates et accessoirement d'hydrates par réaction avec le gaz carbonique et l'humidité de l'atmosphère. Ce sont des substances de composition mal déterminée, et qui sont à la fois solides et insolubles dans l'amine dont elles proviennent.

Dans le tableau qui suit, on décrit l'aspect de quelques compositions cationactives après une exposition d'une durée de 24 heures à l'air libre.

| Produit | Aspect |
|---|---|
| OPDA 2.OE | Inchangé |
| SPDA 2.OP | Inchangé |
| OPDA 2.OE 50 %<br>$iC_{13}$ OPPDA | Inchangé |
| $iC_{13}$ OPPDA 50 % | Inchangé |
| SPDA 1.5 OP | Inchangé |
| N-ALKIZ | Liquide très trouble et précipité |
| OPDAM | Croûte en surface |
| SPDA | Croûte très épaisse en surface et précipité |
| $C_{13}$ OPPDA | Croûte en surface et précipité |
| SPDAM 50 %<br>$iC_{13}$ OPPDA 50 % | Croûte en surface |

On constate ici que la résistance à l'altération à l'atmosphère est la propriété des amines oxyalkylées et des étheramines à chaîne ramifiée, et que les compositions contenant des proportions notables de produits cationactifs instables restent instables à l'atmosphère.

Exemple 3

Homogénéité des formulations aqueuses de composés aminés cationactifs.

L'emploi industriel des amines cationactives impose très généralement leur mise en solution aqueuse. Ces composés étant insolubles dans l'eau, on fait appel à des solutions de sels d'amines, très généralement des chlorhydrates, qui eux-mêmes n'offrent qu'une solubilité très limitée.

Dans le présent exemple, on a réalisé, comme on le fait souvent dans l'industrie des émulsions cationiques de bitume, des solutions titrant 5 kilogrammes du dérivé aminé par $m^3$ de solution, en commençant par disperser la composition cationactive dans l'eau préalablement portée à environ 60°C, en ajoutant ensuite de l'acide chlorhydrique commercial en quantité suffisante pour que le pH se fixe à 2, puis en abandonnant la solution au refroidissement à l'ambiante. On observe les solutions après un repos de 24 heures.

On constate que les alkyldiamines de type SPDA ou OPDA donnent toujours, après refroidissement, des solutions moirées, qui laissent déposer en partie leur matière active sous forme de chlohydrates insolubles ; que la SPDA-2.OP donne des solutions troubles avec léger dépôt. En revanche, les solutions réalisées à partir des compositions selon l'invention ainsi que de leurs composants individuels, à savoir les amines oxyalkylées et les étheramines avec la définition au sens de l'invention que l'on a donnée plus haut, restent toujours limpides et donc absolument homogènes

Exemple 4

Comme on l'a vu dans l'exemple précédent, la pratique industrielle est de disperser le dérivé aminé dans l'eau chaude préalablement à sa neutralisation par un acide.

Certains de ces dérivés aminés présentent dans ces conditions un grave défaut, qui résulte de phénomènes d'hydratation à chaud et qui se manifeste par la formation de produits gommeux, qui ne se dispersent plus dans l'eau chaude, et qui par la suite ne se dissolvent que fort lentement par addition d'acide. On met en évidence ce comportement fort gênant par un essai très simple qui consiste à ajouter à 500 ml d'eau à 60° C disposés dans un vase de Bécher d'une contenance d'un litre, et sous agitation lente, 5 g du dérivé aminé, et à observer l'évolution du système. On a relevé :

- que la SPDA fond en donnant une émulsion instable qui, dès cessation de l'agitation, se résoud en gouttes huileuses qui viennent flotter à la surface ;
- que l'OPDA forme une émulsion instable, au sein de laquelle se rassemblent rapidement des grumeaux d'apparence caoutchouteuse, qui ne se dispersent plus, même sous forte agitation ;
- qu'une composition selon l'invention, telle que constituée de 60% d'OPDA-1,5.OE et 40% de $iC_{13}$OPPDA, donne une émulsion stable qui n'évolue sensiblement plus lorsque l'on poursuit l'opération.

Exemple 5

Préparation à froid de solutions de chlorhydrates de substances alkylaminées comme phase dispersante pour la préparation d'émulsions de bitumes routiers.Dans cet exemple, on expose les résultats obtenus dans la réalisation de solutions de compositions cationactives à la concentration de 5 kg de composé actif par tonne ou $m^3$ de solution ; de telles solutions sont couramment utilisées comme phases dispersantes pour la préparation d'émulsions de bitume routier. Mais ici au lieu de procéder à 60°C comme il est habituel dans cette industrie, on procède en l'absence de tout chauffage : on disperse directement sous agitation douce la composition cationactive dans l'eau prise à la température ambiante, puis on ajoute lentement la quantité convenable d'acide chlorhydrique commercial pour que le pH se fixe à 2.

Avec la SPDA, l'opération est impossible : la diamine ne se disperse pas, et l'introduction ultérieure d'acide chlorhydrique n'est suivie dans l'immédiat d'aucune amélioration de l'hétérogénéité du mélange.

Avec la SPDA-2.OP, on obtient une dispersion acceptable de la composition aminée sous forme d'une émulsion ; après addition d'acide chlorhydrique commercial (d=1,18), à raison de 7,5 litres par $m^3$ de dispersion, le pH se fixe à 2 et on obtient une phase aqueuse apparemment dépourvue de particules solides ou semi-solides, mais néanmoins très trouble.

Avec la N-ALKIZ, la dispersion dans l'eau froide est difficile et incomplète. L'aspect de la masse s'améliore lentement après addition de 11,25 litres d'acide chlorhydrique par $m^3$. On n'obtient une phase aqueuse acceptablement homogène et claire qu'après une vingtaine de minutes d'agitation.

Avec une composition selon l'invention, comprenant 50% d'OPDA-2.OE et 50% de $iC_{13}$-OPPDA, la dispersion dans l'eau froide est immédiate, et après addition de 7,5 litres d'acide chlorhydrique commercial par $m^3$, on obtient de façon pratiquement instantanée une phase aqueuse absolument limpide.

Cette possibilité nouvelle et inattendue d'obtenir à partir des compositions selon l'invention des solutions cationactives prêtes à l'emploi par opérations de dispersion et de salification conduites à froid et dans de très brefs délais constitue un gros avantage industriel.

Exemple 6

Solutions aqueuses concentrées.

Il y a divers avantages incontestables à préparer des solutions aqueuses de dérivés aminés cationactifs les plus concentrées possibles, qui soient et restent homogènes, à plus forte raison, qui ne présentent aucun signe de gélification, et soient de ce fait instantanément pompables, et instantanément diluables à froid.

On montre ici qu'il est possible de réaliser de telles solutions concentrées avec des compositions cationactives selon l'invention. On a visé une solution contenant 125 kg par $m^3$ de solution, de la composition formée de 60% d'OPDA-2.OE et de 40% de $iC_{13}$-OPPDA. A cette fin, on place dans un vase de Becher 50 g de la composition cationactive ci-dessus, puis on y verse 400 ml d'eau à 50°C. Sous agitation douce, on obtient une dispersion homogène quasi instantanée. On ajoute alors 21,5 ml d'acide chlorhydrique commercial (d=1,18), et on obtient immédiatement une solution limpide dont le pH est de 2.

On répète la même opération, cette fois avec de l'eau à température ambiante : on aboutit au même résultat.

Les solutions ainsi obtenues se conservent indéfiniment. On peut les diluer par exemple avec 24 volumes d'eau acidifiée au même pH de 2 : on obtient ainsi très facilement à partir d'une solution de stock, une solution titrant 0,5% de composés cationactifs, qui est d'aspect identique à la solution de composition voisine de l'exemple 5 obtenue par voie directe. A titre de comparaison, si on fabrique des solutions à même concentration de 12,5 % avec de la SPDAM, la solution réalisée à chaud est limpide lors de son retour à la température ambiante ; elle le reste encore 24 heures après sa préparation, mais un dépôt se forme après 48 heures. La solution réalisée à froid rencontre quelques difficultés dues à un début de gélification, qui s'évanouissent dès qu'on ajoute l'acide ; on n'aboutit cependant ainsi qu'à une solution trouble, qui dépose en moins de 24 heures

Exemple 7

Les produits selon l'invention sont utilisables pour la fabrication d'émulsions de bitumes routiers. L'exemple porte sur des émulsions classiques pour revêtements superficiels, titrant 60% de bitume, et que l'on obtient selon un procédé bien connu de l'homme du métier qui consiste à forcer ensemble dans un turbomalaxeur le bitume à une température de l'ordre de 140°C et une phase dispersante constituée d'une solution chlorhydrique d'un émulsifiant convenablement choisi.

On s'intéresse aux caractéristiques suivantes de l'émulsion obtenue :

- pH ;
- viscosité (en mm2/s, mesurée selon le norme AFNOR T 66-020) ;
- retenue au tamis de 0,630 mm, exprimée en pour mille par rapport à l'émulsion (selon la norme du Laboratoire Central des Ponts et Chaussées, RLE-AC.2.1965) ;
- diamètre moyen des particules de bitume en micromètre (mesure au granulomètre à laser, appareil SILAS, modèle 715) ;
- la stockabilité à 7 jours (mesurée par la différence de la teneur en bitume % entre deux prélèvements effectués respectivement en surface et au fond du vase de stockage, selon la norme AFNOR T 66-022) ;
- l'indice de rupture (selon la norme AFNOR T 66-017) ;
- l'adhésivité sur gravillons siliceux (selon la norme AFNOR T 66-018).

On prend pour références les émulsions de bitume de pénétration 180/220, réalisées à l'aide d'une phase aqueuse de SPDA neutralisée à pH2 par l'acide chlorhydrique, et titrant 600 kg de bitume et respectivement 1,5 et 2 kg de SPDA par tonne d'émulsion. Ces émulsions sont des produits de fabrication très courante. Des fabrications de laboratoire ont les caractéristiques suivantes :

| Emulsifiant<br>Kg / tonne d'émulsion | SPDA<br>1.5 | SPDA<br>2 |
|---|---|---|
| pH d'émulsion | 2 | 2 |
| Tamisage à 0,60 mm - retenue %. | 0 | 0 |
| Viscosité ($mm^2/s$) | 34 | 25 |
| Stockabilité à 7 jours % | 12 | 10 |
| Diamètre moyen ($\mu m$) | 5,6 | 2,6 |
| Indice de rupture | 158 | 164 |
| Adhésivité | 100 | 100 |

Des émulsions réalisées avec des compositions telles que par
$C_1$ = 60% d'OPDA-2.OE + 40% de $iC_{13}$-OPPDA, et
$C_2$ = 50% d'OPDA-2.OE + 50% de $iC_{13}$-OPPDA, montrent à la fois qu'il est possible de réaliser à l'aide de compositions selon l'invention de correctes émulsions bitumineuses pour répandage et que les propriétés cationactives de telles compositions se comparent excellemment à celles des alkylproprylènes diamines traditionnelles.
Les caractéristiques de ces émulsions figurent au tableau ci-après.

| Emulsifiant<br>Kg / tonne d'émulsion | $C_1$<br>1.5 | $C_2$<br>1.5 |
|---|---|---|
| pH d'émulsion | 2 | 2 |
| Tamisage à 0,60 mm - retenue %. | 0,15 | 0,05 |
| Viscosité ($mm^2/s$) | 46 | 41 |
| Stockabilité à 7 jours % | 13 | 17 |
| Diamètre moyen (μm) | 6,6 | 5,8 |
| Indice de rupture | 132 | 139 |
| Adhésivité | 100 | 100 |

Exemple 8

On procède comme dans l'exemple 7 à la comparaison d'émulsions réalisées avec de la SPDA et avec la composition $C_1$ selon l'invention, mais à la différence de l'exemple précédent, on met en oeuvre des phases aqueuses qui ont été laissées au repos pendant 48 heures et dont on n'a prélevé que la partie surnageante. On simule ainsi une fabrication industrielle faite à partir de phases aqueuses préparées à l'avance et qui auraient pu vieillir par séparation de sels insolubles de l'émulsifiant.

Les résultats sont consignés dans le tableau ci-après.

| Emulsifiant<br>Kg / tonne d'émulsion | SPDA<br>(1.5) | $C_1$<br>(1.5) |
|---|---|---|
| pH d'émulsion | 2 | 2 |
| Tamisage à 0,60 mm - retenue %. | 2 | 0,06 |
| Viscosité ($mm^2/s$) | 22 | 40 |
| Stockabilité à 7 jours % | 25 | 16 |
| Diamètre moyen (μm) | 14 | 6,1 |
| Indice de rupture | 80 | 135 |
| Adhésivité | 95 | 100 |

On observe que l'émulsion obtenue avec le produit selon l'invention est pratiquement identique à celle de l'exemple 7. En revanche, la fabrication de l'émulsion à la SPDA a été fortement perturbée, et l'émulsion produite présente des signes manifestes d'instabilité : retenue importante à 0,63 mm, une forte décantation au stockage avec traces de rupture, un fort diamètre moyen, et un faible indice de rupture, qui sont autant d'indications que la phase aqueuse s'est fortement appauvrie en matière active par sédimentation.

Exemple 9

On a effectué à titre de comparaison une émulsion de bitume dur, de pénétration 40/50. Les exigences habituelles pour émulsionner ce genre de bitume imposent qu'il soit porté à une température de 160 °C. Dans ces conditions, pour pouvoir utiliser les phases aqueuses ordinaires aux températures habituellement utilisées, soit environ 55°C, il est obligatoire d'émulsionner sous pression de 2 bars, sous peine d'incidents dans le moulin colloïdal même, par ébullition de l'émulsion en formation.
La fabrication de l'émulsion à l'aide d'un équipement travaillant à pression atmosphérique exige que la phase aqueuse soit au maximum à la température de 30° C. La tentative réalisée en ce sens avec une phase aqueuse faite avec de la SPDA échoue totalement. En revanche, l'émulsion réalisée avec la composition $C_1$ selon l'invention fournit une émulsion de bonnes caractéristiques, comparables à celles obtenues à partir de SPDA sous pression. Les résultats en sont présentés ci-après.

| Emulsifiant<br>Kg / tonne d'émulsion | SPDA<br>1,8 | $C_1$<br>1,8 |
|---|---|---|
| pH d'émulsion | 2,5 | 2,5 |
| Tamisage à 0,60 mm - retenue %. | 0 | 0 |
| Viscosité ($mm^2/s$) | 27 | 29 |
| Stockabilité à 7 jours % | 10 | 13 |
| Diamètre moyen (µm) | 3,4 | 5 |
| Indice de rupture | 175 | 140 |
| Adhésivité | 100 | 100 |

Exemple 10

On fabrique une émulsion avec une phase aqueuse obtenue par dilution d'une préparation concentrée. On simule ici des conditions de préparation difficile d'émulsions dans des unités où les possibilités de préparation des émulsifiants dans leur forme d'emploi sont limitées à de simples dilutions de préparations extemporanées approvisionnées sous forme concentrée. L'émulsion visée est une émulsion à 60% de bitume turbinée dans les conditions habituelles, bitume 180-220 à 135°C pour 60%, et phase aqueuse à 55°C à 0,5% de composition C1 faite, soit normalement par dissolution de la quantité nécessaire de la composition cationactive dans l'eau et acidification, soit par dilution d'une composition concentrée préparée comme dans l'exemple 6, et reprise sept jours plus tard.
On constate ci-après que les résultats obtenus selon l'une ou l'autre méthode sont très équivalents. La méthode de déconcentration du savon est, il va sans dire, extrèmement avantageuse pour la fabrication d'émulsions à l'aide d'unités mobiles ou dans des installations situées sur des sites déshérités.

| Emulsifiant<br>Kg / tonne d'émulsion | $C_1$<br>2 | $C_1$<br>2 |
|---|---|---|
| Type de phase aqueuse | Standard | Décon-centrée |
| pH d'émulsion | 2,3 | 2,2 |
| Tamisage à 0,60 mm - retenue %. | 0 | 0 |
| Viscosité ($mm^2/s$) | 46 | 42 |
| Diamètre moyen (µm) | 6,0 | 5,9 |
| Indice de rupture | 140 | 140 |
| Adhésivité | 100 | 100 |

Exemple 11

On simule une fabrication à l'aide d'une phase aqueuse réalisée dans une installation où il est difficile de chauffer à basse température : on a opté pour la fabrication et la réalisation à froid de l'émulsifiant.
On compare à cet effet une émulsion à 0,2% de la composition $C_2$ telle que réalisée à froid dans l'exemple 5 et utilisée de même à froid, le bitume restant comme précédemment utilisé à sa température normale de 135°, et une émulsion de même compsition réalisée dans les conditions standards.

| Emulsifiant<br>Kg / tonne d'émulsion | $C_2$<br>2 | $C_2$<br>2 |
|---|---|---|
| Température phase aqueuse | 19° C | 55° C |
| pH d'émulsion | 2,3 | 2,2 |
| Tamisage à 0,60 mm - retenue %. | 0 | 0,15 |
| Viscosité ($mm^2/s$) | 46 | 41,5 |
| Diamètre moyen (µm) | 6,0 | 5,8 |
| Indice de rupture | 140 | 145 |
| Adhésivité | 100 | 100 |

On constate que l'opération réalisée dans ces conditions, fort inhabituelles mais très séduisantes par leur commodité, de basse température de la phase aqueuse conduit à une émulsion de répandage de qualité tout-à-fait acceptable.

## Revendications

1. Compositions de dérivés cationactifs aminés non inflammables, absolument liquides à température ambiante, qui conservent intégralement leur liquidité lorsqu'elles restent indéfiniment exposées à l'atmosphère, qui ne donnent pas lieu à formation de gels, de précipités ou de dépôts lorsqu'elles ont été maintenues de façon prolongée à des températures voisines de zéro degré centigrade ou qui ne donnent pas lieu à formation de tels gels, précipités ou dépôts persistant longtemps par retour à la température ambiante,
caractérisées en ce qu'elles contiennent de 20 à 80% en poids d'alkylétheramines de formule générale
$$R\text{-}O\text{-}(CH_2)_n\text{-}[\text{-}NH\text{-}(CH_2)_m\text{-}]_p\text{-}NH_2$$
où R est une chaîne alkyle ramifiée, du type

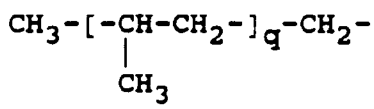

avec q valant de 2 à 5,
où n est un nombre entier égal à 2 ou 3,
où m est un nombre entier égal à 2 ou 3,
où p est un nombre pouvant prendre toutes valeurs de 0 à 3,
et
de 80 à 20% en poids d'amines ou polyamines grasses oxyalkylées selon la formule générale :

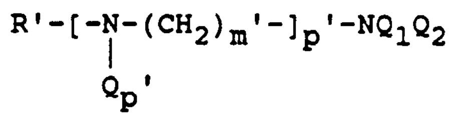

où R′ est une chaîne hydrocarbonée linéaire, saturée ou insaturée, comprenant de 10 à 22 atomes de carbone,
où m′ est un nombre entier égal à 2 ou 3,
où p′ est un nombre pouvant prendre toutes valeurs de 0 à 3,
où $Q_p'$, $Q_1$, $Q_2$ sont l'hydrogène ou un groupe hydroxyéthyle $-CH_2-CH_2-OH$ ou un groupe hydroxyéthyloxyéthyle $-CH_2-CH_2-O-CH_2-CH_2-OH$, identiques ou différents, sous la seule condition que ce ne soient pas tous des hydrogènes.

2. Compositions selon la revendication 1 caractérisées en ce les alkylétheramines sont des N-alkyloxypropyl-propylènediamines répondant à la formule générale
$$R\text{-}O\text{-}CH_2\text{-}CH_2\text{-}CH_2\text{-}NH\text{-}CH_2\text{-}CH_2\text{-}CH_2\text{-}NH_2$$

3. Compositions selon la revendication 2 caractérisées en ce que les polyamines grasses oxyalkylées sont des alkylpropylène diamines oxyalkylées selon la formule générale :
$$R'\text{-}NQ_3\text{-}CH_2\text{-}CH_2\text{-}CH_2\text{-}NQ_1Q_2$$
où $Q_1$, $Q_2$, $Q_3$ sont l'hydrogène ou un groupe hydroxyéthyle $-CH_2-CH_2-OH$ ou encore un groupe hydroxyéthyloxyéthyle $-CH_2-CH_2-O-CH_2-CH_2-OH$, identiques ou différents, sous la seule condition que ce ne soient pas tous des hydrogènes.

4. Composition selon la revendication 1 caractérisée en ce qu'elle comprend de 20 à 80% en poids de N-isotridécyloxypropyl-propylènediamine et de 80 à 20% en poids d'oléylpropylènediamine dioxyéthylée.

5. Procédé pour l'obtention d'émulsions de bitume routier dans lequel on fait passer dans un turbomalaxeur un bitume fondu et une phase dispersante constituée d'une composition aqueuse émulsifiante, caractérisée en ce que la composition aqueuse contient une composition cationactive constituée de 20 à 80% en poids d'alkylétheramines de formule générale
$$R\text{-}O\text{-}(CH_2)_n\text{-}[\text{-}NH\text{-}(CH_2)_m\text{-}]_p\text{-}NH_2$$
où R est une chaîne alkyle ramifiée, du type

$$CH_3-[-\underset{\displaystyle CH_3}{\underset{|}{C}H}-CH_2-]_q-CH_2-$$

avec q valant de 2 à 5,
où n est un nombre entier égal à 2 ou 3,
où m est un nombre entier égal à 2 ou 3,
où p est un nombre pouvant prendre toutes valeurs de 0 à 3,
et
de 80 à 20 % en poids d'amines ou polyamines grasses oxyalkylées selon la formule générale :

$$R'-[-\underset{\displaystyle Q_p'}{\underset{|}{N}}-(CH_2)_{m'}-]_{p'}-NQ_1Q_2$$

où R' est une chaîne hydrocarbonée linéaire, saturée ou insaturée, comprenant de 10 à 22 atomes de carbone,
où m' est un nombre entier égal à 2 ou 3,
où p' est un nombre pouvant prendre toutes valeurs de 0 à 3,
où $Q_p'$, $Q_1$, $Q_2$ sont l'hydrogène ou un groupe hydroxyéthyle $-CH_2-CH_2-OH$ ou un groupe hydroxyéthyloxyéthyle $-CH_2-CH_2-O-CH_2-CH_2-OH$, identiques ou différents, sous la seule condition que ce ne soient pas tous des hydrogènes.

6. Procédé d'obtention d'émulsions bitumineuses selon la revendication 5 caractérisé en ce que les alkylétheramines sont des N-alkyloxypropyl-propylènediamines répondant à la formule générale

$$R-O-CH_2-CH_2-CH_2-NH-CH_2-CH_2-CH_2-NH_2$$

7. Procédé d'obtention d'émulsions bitumineuses selon la revendication 5 caractérisé en ce que que les polyamines grasses oxyalkylées sont des alkylpropylène diamines oxyalkylées selon la formule générale :

$$R'-NQ_3-CH_2-CH_2-CH_2-NQ_1Q_2$$

où $Q_1$, $Q_2$, $Q_3$ sont l'hydrogène ou un groupe hydroxyéthyle $-CH_2-CH_2-OH$ ou encore un groupe hydroxyéthyloxyéthyle $-CH_2-CH_2-O-CH_2-CH_2-OH$, identiques ou différents, sous la seule condition que ce ne soient pas tous des hydrogènes.

8. Procédé d'obtention d'émulsions bitumineuses selon les revendications 5, 6 ou 7 caractérisé en ce que la phase aqueuse émulsionnante est mise en oeuvre à la température ambiante.

9. Procédé d'obtention d'émulsions bitumineuses selon les revendications 5, 6 ou 7, caractérisé en ce que la phase aqueuse est préalablement préparée à une concentration en composition aminée cationactive supérieure à 100 kg par m3, puis diluée à la concentration d'usage au moment de l'emploi.

10. Procédé d'obtention d'émulsions bitumineuses selon les revendications 5, 7, 8 et 9, caractérisé en ce que la phase aqueuse émulsionnante contient une composition constituée de 20 à 80% en poids de N-isotridécyloxypropyl-propylènediamine et de 80 à 20% en poids d'oléylpropylènediamine dioxyéthylée.

## Claims

1. Compositions of nonflammable cation-active amine derivatives, completely liquid at room temperature, which fully preserve their liquidity when they remain indefinitely exposed to the atmosphere, which do not give rise to the formation of gels, precipitates or deposits when they have been maintained for a long time at temperatures in the region of zero degrees centigrade or which do not give rise to the formation of such gels, precipitates or deposits which persist for a long time when brought back to room temperature, characterised in that they contain from 20 to 80% by weight of alkyl ether amines of general formula

$$R\text{-}O\text{-}(CH_2)_n\text{-}[\text{-}NH\text{-}(CH_2)_m\text{-}]_p\text{-}NH_2$$

where R is a branched alkyl chain, of the type

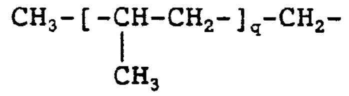

with q having a value from 2 to 5,
where n is an integer equal to 2 or 3,
where m is an integer equal to 2 or 3,
where p is a number which is able to take all values iron 0 to 3,
and
from 80 to 20% by weight of oxyalkylated fatty amines or polyamines according to the general formula:

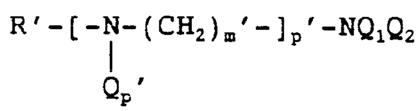

where R′ is a saturated or unsaturated linear hydrocarbon chain, comprising from 10 to 22 carbon atoms,
where m′ is an integer equal to 2 or 3,
where p′ is a number which is able to take all values from 0 to 3,
where $Q_p'$, $Q_1$ and $Q_2$ are hydrogen or a hydroxyethyl group $-CH_2\text{-}CH_2\text{-}OH$ or a hydroxyethyloxyethyl group $-CH_2\text{-}CH_2\text{-}O\text{-}CH_2\text{-}CH_2\text{-}OH$, which are identical or different, with the sole condition that they are not all hydrogens.

2. Compositions according to Claim 1, characterised in that the alkyl ether amines are N-(alkyloxypropyl)propylenediamines corresponding to the general formula

$$R\text{-}O\text{-}CH_2\text{-}CH_2\text{-}CH_2\text{-}NH\text{-}CH_2\text{-}CH_2\text{-}CH_2\text{-}NH_2$$

3. Compositions according to Claim 2, characterised in that the oxyalkylated fatty polyamines are oxyalkylated alkylpropylenediamines according to the general formula:

$$R'\text{-}NQ_3\text{-}CH_2\text{-}CH_2\text{-}CH_2\text{-}NQ_1Q_2$$

where $Q_1$, $Q_2$ and $Q_3$ are hydrogen or a hydroxyethyl group $-CH_2\text{-}CH_2\text{-}OH$ or alternatively a hydroxyethyloxyethyl group $-CH_2\text{-}CH_2\text{-}O\text{-}CH_2\text{-}CH_2\text{-}OH$, which are identical or different, with the sole condition that they are not all hydrogens.

4. Composition according to Claim 1, characterised in that it comprises from 20 to 80% by weight of N-(isotridecyloxypropyl)propylenediamine and from 80 to 20% by weight of dioxyethylated oleylpropylenediamine.

5. Process for producing road asphalt emulsions, in which a molten asphalt and a dispersing phase consisting of an emulsifying aqueous composition are passed into a turbine mixer, characterised in that the aqueous composition contains a cation-active composition consisting of 20 to 80% by weight of alkyl ether amines of general formula

$$R\text{-}O\text{-}(CH_2)_n\text{-}[NH\text{-}(CH_2)_m\text{-}]_p\text{-}NH_2$$

where R is a branched alkyl chain, of the type

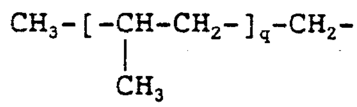

with q having a value from 2 to 5,
where n is an integer equal to 2 or 3,
where m is an integer equal to 2 or 3,
where p is a number which is able to take all values from 0 to 3,

and
from 80 to 20% by weight of oxyalkylated fatty amines or polyamines according to the general formula:

$$R'-[-\underset{\displaystyle Q_p'}{\underset{|}{N}}-(CH_2)_{m'}-]_{p'}-NQ_1Q_2$$

where R′ is a saturated or unsaturated linear hydrocarbon chain, comprising from 10 to 22 carbon atoms,
where m′ is an integer equal to 2 or 3,
where p′ is a number which is able to take all values from 0 to 3,
where $Q_p'$, $Q_1$ and $Q_2$ are hydrogen or a hydroxyethyl group $-CH_2-CH_2-OH$ or a hydroxyethyloxyethyl group $-CH_2-CH_2-O-CH_2-CH_2-OH$, which are identical or different, with the sole condition that they are not all hydrogens.

6. Process for producing bituminous emulsions according to Claim 5, characterised in that the alkyl ether amines are N-(alkyloxypropyl)propylenediamines corresponding to the general formula

$$R-O-CH_2-CH_2-CH_2-NH-CH_2-CH_2-CH_2-NH_2$$

7. Process for producing bituminous emulsions according to Claim 5, characterised in that the oxyalkylated fatty polyamines are oxyalkylated alkylpropylenediamines according to the general formula:

$$R'-NQ_3-CH_2-CH_2-CH_2-NQ_1Q_2$$

where $Q_1$, $Q_2$ and $Q_3$ are hydrogen or a hydroxyethyl group $-CH_2-CH_2-OH$ or alternatively a hydroxyethyloxyethyl group $-CH_2-CH_2-O-CH_2-CH_2-OH$, which are identical or different, with the sole condition that they are not all hydrogens.

8. Process for producing bituminous emulsions according to Claims 5, 6 or 7, characterised in that the emulsifying aqueous phase is used at room temperature.

9. Process for producing bituminous emulsions according to Claims 5, 6 or 7, characterised in that the aqueous phase is prepared beforehand with a concentration of cation-active amine composition which is greater than 100 kg per $m^3$ and is then diluted to the use concentration at the time of use.

10. Process for producing bituminous emulsions according to Claims 5, 7, 8 and 9, characterised in that the emulsifying aqueous phase contains a composition consisting of 20 to 80% by weight of N-(isotridecyloxypropyl)propylenediamine and 80 to 20% by weight of dioxyethylated oleylpropylenediamine.

## Patentansprüche

1. Mischungen von kationaktiven, aminierten, nicht entflammbaren Derivaten bei Raumtemperatur völlig flüssigen, die ihre Flüssigkeit uneingeschränkt beibehalten, wenn sie unbegrenzt der Umgebung ausgesetzt sind, die keine Bildung von Gelen, Niederschlägen oder Absonderungen ergeben, wenn sie längere Zeit Temperaturen um null Grad Celsius ausgesetzt wurden oder die keine Bildung solcher Gele, Niederschläge oder Absonderung ergeben, die nach Rückkehr zu Raumtemperatur länger beständig sind, dadurch gekennzeichnet, daß diese 20 bis 80 Gewichtsprozent Aminoalkylether der allgemeinen Formel

$$R-O-(CH_2)_n-[-NH-(CH_2)_m-]_p-NH_2$$

enthalten, wobei R eine verzweigte Alkylkette des Typs

$$CH_3-[-\underset{\displaystyle CH_3}{\underset{|}{CH}}-CH_2-]_q-CH_2-$$

ist, mit Werten für q von 2 bis 5,
n eine ganze Zahl gleich 2 oder 3 ist,
m eine ganze Zahl gleich 2 oder 3 ist,
p eine Zahl, die alle Werte zwischen 0 und 3 annehmen kann und

80 bis 20 Gewichtsprozent gegebenenfalls alkoxylierte Fettamine oder -polyamine der allgemeinen Formel:

$$R'-[-\underset{Q_{p'}}{\underset{|}{N}}-(CH_2)_{m'}-]_{p'}-NQ_1Q_2$$

wobei R′ eine lineare Kohlenwasserstoffkette ist, gesättigt oder ungesättigt, die 10 bis 22 Kohlenstoffatome enthalten kann,
m′ eine ganze Zahl gleich 2 oder 3 ist,
p′ eine Zahl, die jeden Wert zwischen 0 und 3 annehmen kann,
$Q_{p'}$, $Q_1$, $Q_2$ Wasserstoff oder eine Hydroxyethylgruppe $-CH_2-CH_2-OH$ oder eine β-Hydroxyethylethergruppe $-CH_2-CH_2-O-CH_2-CH_2-OH$ sind, identisch oder verschieden, unter der einzigen Bedingung, daß sie nicht alle Wasserstoff sind.

**2.** Mischungen nach Anspruch 1, dadurch gekennzeichnet, daß die Aminoalkylether N-Alkoxypropyl-propylendiamine sind, die der allgemeinen Formel

$$R-O-CH_2-CH_2-CH_2-NH-CH_2-CH_2-CH_2-NH_2$$

entsprechen.

**3.** Mischungen nach Anspruch 2, dadurch gekennzeichnet, daß die alkoxylierten Fettpolyamine alkoxylierte Alkylpropylendiamine sind nach der allgemeinen Formel:

$$R'-NQ_3-CH_2-CH_2-CH_2-NQ_1Q_2$$

wobei $Q_1$, $Q_2$, $Q_3$ Wasserstoff oder eine Hydroxyethylgruppe $-CH_2-CH_2-OH$ oder auch eine β-Hydroxyethylethergruppe $-CH_2-CH_2-O-CH_2-CH_2-OH$ sind, identisch oder verschieden, unter der einzigen Bedingung, daß sie nicht alle Wasserstoff sind.

**4.** Mischung nach Anspruch 1, dadurch gekennzeichnet, daß sie 20 bis 80 Gewichtsprozent N-Isotridecyloxypropylpropylendiamin und 80 bis 20 Gewichtsprozent dioxyethyliertes Oleylpropylendiamin beinhaltet.

**5.** Verfahren zur Gewinnung von Straßenbitumen, bei dem man geschmolzenes Bitumen und eine aus einer wässrigen Emulsionsmischung bestehende dispergierende Phase durch einen Turbomischer leitet, dadurch gekennzeichnet, daß die wässrige Mischung eine kationaktive Mischung enthält, die zu 20 bis 80 Gewichtsprozent aus Aminoalkylethern der allgemeinen Formel

$$R-O-(CH_2)_n-[-NH-(CH_2)_m-]_p-NH_2$$

in der R eine verzweigte Alkylkette des Typs

$$CH_3-[-\underset{CH_3}{\underset{|}{CH}}-CH_2-]_q-CH_2-$$

ist,
mit Werten für q von 2 bis 5,
n eine ganze Zahl gleich 2 oder 3 ist,
m eine ganze Zahl gleich 2 oder 3 ist,
p eine Zahl, die alle Werte von 0 bis 3 annehmen kann,
und
zu 80 bis 20 Gewichtsprozent aus alkoxylierten Fettaminen oder -polyaminen der allgemeinen Formel besteht:

$$R'-[-\underset{Q_{p'}}{\underset{|}{N}}-(CH_2)_{m'}-]_{p'}-NQ_1Q_2$$

wobei R′ eine gesättigte oder ungesättigte lineare Kohlenwasserstoffkette mit 10 bis 22 Kohlenstoffatomen ist,
m′ eine ganze Zahl gleich 2 oder 3 ist,
p′ eine Zahl, die alle Werte zwischen 0 und 3 annehmen kann,
$Q_{p'}$, $Q_1$, $Q_2$ gleich oder verschieden Wasserstoff oder eine Hydroxyethylgruppe $-CH_2-CH_2-OH$ oder eine β-Hydroxyethylethergruppe $-CH_2-CH_2-O-CH_2-CH_2-OH$ sind, unter der einzigen Bedingung, daß sie nicht alle Wasserstoff sind.

6. Verfahren zur Gewinnung von bituminösen Emulsionen nach Anspruch 5, dadurch gekennzeichnet, daß die Aminoalkylether N-Alkoxypropyl-propylendiamine der allgemeinen Formel

$$R-O-CH_2-CH_2-CH_2-NH-CH_2-CH_2-CH_2-NH_2$$

entsprechen.

7. Verfahren zur Gewinnung von bituminösen Emulsionen nach Anspruch 5, dadurch gekennzeichnet, daß die stark alkoxylierten Polyfettamine alkoxylierte Alkylpropylendiamine der allgemeinen Formel sind:

$$R'-NQ_3-CH_2-CH_2-CH_2-NQ_1Q2$$

in der $Q_1$, $Q_2$, $Q_3$ gleich oder verschieden Wasserstoff, eine Hydroxyethylgruppe $-CH_2-CH_2-OH$ oder eine β-Hydroxyethylethergruppe $-CH_2-CH_2-O-CH_2-CH_2-OH$ sind, unter der einzigen Bedingung, daß sie nicht alle Wasserstoff sind.

8. Verfahren zur Gewinnung von bituminösen Emulsionen nach den Ansprüchen 5, 6 oder 7, dadurch gekennzeichnet, daß die wässrige, emulgierende Phase bei Raumtemperatur eingesetzt wird.

9. Verfahren zur Gewinnung von bituminösen Emulsionen nach den Ansprüchen 5, 6 oder 7, dadurch gekennzeichnet, daß die wässrige Phase vorher mit einer aminierten, kationaktiven Mischung bei einer Konzentration über 100 kg pro $m^3$ hergestellt wird, und dann zum Zeitpunkt der Benutzung auf die Gebrauchskonzentration verdünnt wird.

10. Verfahren zur Gewinnung von bituminösen Emulsionen nach den Ansprüchen 5, 7, 8 und 9, dadurch gekennzeichnet, daß die wässrige, emulgierende Phase eine Mischung enthält, die zu 20 bis 80 Gewichtsprozent aus N-Isotridecyloxypropyl-propylendiamin und zu 80 bis 20 Gewichtsprozent aus dioxyethyliertem Oleylpropylendiamin besteht.